(19) Europäisches Patentamt
European Patent Office
Office européen des brevets

(11) EP 4 617 658 A1

(12) **EUROPÄISCHE PATENTANMELDUNG**

(43) Veröffentlichungstag:
17.09.2025 Patentblatt 2025/38

(21) Anmeldenummer: 25161205.7

(22) Anmeldetag: 03.03.2025

(51) Internationale Patentklassifikation (IPC):
G01N 33/00 (2006.01)    G01N 33/497 (2006.01)

(52) Gemeinsame Patentklassifikation (CPC):
G01N 33/4972; G01N 33/007; G01N 33/0063

(84) Benannte Vertragsstaaten:
AL AT BE BG CH CY CZ DE DK EE ES FI FR GB GR HR HU IE IS IT LI LT LU LV MC ME MK MT NL NO PL PT RO RS SE SI SK SM TR
Benannte Erstreckungsstaaten:
BA
Benannte Validierungsstaaten:
GE KH MA MD TN

(30) Priorität: 11.03.2024 DE 102024106856

(71) Anmelder: Dräger Safety AG & Co. KGaA
23560 Lübeck (DE)

(72) Erfinder:
• Mattern-Frühwald, Marie-Isabell
23558 Lübeck (DE)
• Baesler, Malte
23558 Lübeck (DE)

(54) **VERFAHREN ZUM BETREIBEN EINES GASMESSSYSTEMS UND ENTSPRECHENDES GASMESSSYSTEM**

(57) Es wird ein Verfahren zum Betreiben eines Gasmesssystems bereitgestellt, das Verfahren aufweisend die Schritte: Lagern eines Gasmessgeräts in einer Lagerungsumgebung in einem ersten Betriebszustand, Bestimmen einer Konzentration eines Schadgases in der Lagerungsumgebung durch eine Auswerteeinheit, Bestimmen einer Konzentration des Zielgases in einer Messumgebung durch die Auswerteeinheit in einem zweiten Betriebszustand unter Berücksichtigung der Konzentration des Schadgases und Ausgeben der Konzentration des Zielgases, und/oder Vergleichen einer mit der Konzentration des Schadgases korrelierenden Information mit einem vorbestimmten Schwellenwert durch die Auswerteeinheit und Ausgeben einer Warnung in einem dritten Betriebszustand, wenn die korrelierende Information den vorbestimmten Schwellenwert überschreitet. Ferner wird ein entsprechendes Gasmesssystem bereitgestellt.

Fig. 1

EP 4 617 658 A1

**Beschreibung**

Die vorliegende Erfindung betrifft ein Verfahren zum Betreiben eines Gasmesssystems und ein entsprechendes Gasmesssystem.

Gasmessgeräte mit einem oder mehreren Gassensoren, insbesondere mit einem oder mehreren elektrochemischen Gassensoren, wie elektrochemische Alkoholsensoren, können durch eine Lagerung in einer Schadgas-haltigen Umgebung (Lagerungsumgebung) reversibel oder irreversibel geschädigt werden, so dass deren Messverhalten beeinflusst wird.

Ein Beispiel für ein solches Gasmessgerät ist ein Alkoholmessgerät. Bei einem Gasmessgerät insbesondere bei einem Alkoholmessgerät, kann, beispielsweise bei einer längerfristigen Lagerung (z.B. von einem oder mehreren Tagen und/oder Wochen) in einer Lagerungsumgebung mit beispielsweise Alkoholen als Schadgas eine Reduzierung einer Empfindlichkeit des Gassensors eintreten. Diese Reduzierung kann auch nach Entfernen des Gasmessgeräts aus der Lagerungsumgebung noch mehre Tage anhalten. Durch das Lagern in einer Umgebung mit einem Schadgas wird daher ein Messverhalten des Gasmessgeräts beeinträchtigt. Ein Benutzer des Gasmessgeräts kann jedoch nicht bestimmen, ob eine solche Beeinträchtigung vorliegt.

Ein Alkohol als Schadgas kann beispielsweise durch den Gebrauch von Desinfektionsmitteln (z.B. Handdesinfektionsmittel) oder anderen Lösungsmitteln in der Lagerungsumgebung auftreten. Auch die Aufbewahrung von Desinfektionsmittelgebinden oder Lösungsmittelgebinden in der Lagerungsumgebung kann zu einer Freisetzung von Schadgas wie Alkohol führen.

Von der vorbeschriebenen Problematik des Auftretens von Schadgasen und der Beeinflussung des Messverhaltens des Gasmessgeräts sind insbesondere Gasmessgeräte betroffen, die diffusionsoffen zur Umgebung sind, wie es beispielsweise bei vielen derzeitig kommerziell erhältlichen marktüblichen Alkoholmessgeräten der Fall ist.

Eine Lösung der vorstehenden Problematik ist derzeit nicht bekannt. Aus der DE 10 2022 108 432 A1 ist lediglich bekannt, dass das Problem umgangen werden kann, indem ein Sensor eines Gasmessgeräts während der Lagerung luftdicht verschlossen wird, sodass die potenziell schädliche Lagerungsumgebung nicht auf den Sensor einwirken kann. Diese Methode erfordert somit eine aufwendige und teure mechanische Kapselung des Sensors.

Aus US 2024 / 0 013 647 A1 ist ein System und Verfahren zur Gaserfassung bei gleichzeitiger Korrektur eines Giftpegels (poison level) eines Gassensors bekannt. Der Gassensor umfasst ein Gassensormaterial, das so konfiguriert ist, dass es mit einer Fluidprobe in Kontakt kommt; und eine Messschaltung, die eingerichtet ist, eine erste und eine zweite dielektrische Anregung des Gassensormaterials bei einem ersten bzw. einem zweiten Satz von Frequenzen bereitzustellen, während das Gassensormaterial in Kontakt mit der Flüssigkeitsprobe steht. Auf der Grundlage einer gemessenen Reaktionen des Gassensormaterials auf die erste und zweite dielektrische Anregung bei den ersten und zweiten jeweiligen Frequenzsätzen wird eine Korrektur für einen Giftpegel des Gassensormaterials bereitgestellt.

Es ist daher eine Aufgabe der vorliegenden Erfindung, ein Verfahren zum Betreiben eines Gasmesssystems und ein entsprechendes Gasmesssystem bereitzustellen, das die vorstehend genannten Nachteile nicht oder nur in reduziertem Maße aufweist.

Erfindungsgemäß wird insofern ein Verfahren zum Betreiben eines Gasmesssystems bereitgestellt, das Verfahren aufweisend die Schritte: Lagern eines Gasmessgeräts in einer Lagerungsumgebung in einem ersten Betriebszustand, Bestimmen einer Konzentration eines Schadgases in der Lagerungsumgebung durch eine Auswerteeinheit, Bestimmen einer Konzentration des Zielgases in einer Messumgebung durch die Auswerteeinheit in einem zweiten Betriebszustand unter Berücksichtigung der Konzentration des Schadgases und Ausgeben der Konzentration des Zielgases, und/oder Vergleichen einer mit der Konzentration des Schadgases korrelierenden Information mit einem vorbestimmten Schwellenwert durch die Auswerteeinheit und Ausgeben einer Warnung in einem dritten Betriebszustand, wenn die korrelierende Information den vorbestimmten Schwellenwert überschreitet.

Auf diese Weise kann der Einfluss der Lagerungsumgebung auf das Messverhalten des Gasmessgeräts erkannt und ein Benutzer des Gasmessgeräts durch (sofortige oder verzögerte, d.h. nachgelagerte) Ausgabe der Warnung gewarnt werden, dass das Messverhalten verschlechtert ist. Ergänzend oder alternativ zu der Ausgabe der Warnung kann der Einfluss der Konzentration und/oder Menge des Schadgases auf die berechnete Konzentration des Zielgases zumindest teilweise kompensiert werden, um eine entsprechend korrigierte Konzentration des Zielgases auszugeben. In beiden Fällen ist es somit möglich, einen (qualitativen und/oder quantitativen) Einfluss der Lagerungsumgebung auf das Messverhalten des Gasmessgeräts festzustellen und beim Betrieb des Gasmessgeräts zu berücksichtigen.

Unter einer Lagerungsumgebung wird eine Umgebung verstanden, in welcher ein Gasmessgerät in einem ersten Betriebszustand aufbewahrt werden kann und in welcher ein Schadgas vorhanden sein kann.

Der erste Betriebszustand ist ein Betriebszustand des Gasmessgeräts, in dem eine Messung einer Konzentration des Schadgases stattfindet.

Der zweite Betriebszustand ist ein Betriebszustand des Gasmessgeräts, in dem eine Messung einer Konzentration des Zielgases stattfindet.

Der dritte Betriebszustand ist ein Betriebszustand des Gasmessgeräts, in dem eine Warnung an einen Benutzer des Gasmessgeräts ausgegeben wird.

Der erste Betriebszustand und der dritte Betriebszustand können gleichzeitig oder zeitlich versetzt vorliegen. Der zweite Betriebszustand und der dritte Betriebszustand können gleichzeitig oder zeitlich versetzt vorliegen. Der erste Betriebszustand und der zweite Betriebszustand liegen zeitlich versetzt vor.

Unter einem Schadgas wird eine Substanz verstanden, welche ein Messverhalten des Gasmessgeräts, insbesondere durch andauernde Exposition, beeinflussen kann. Dabei kann es sich um ein Zielgas oder um eine hiervon verschiedene Substanz handeln. Beispielsweise kann es sich bei dem Schadgas um ein Alkohol (z.B. Ethanol, Methanol, 1-(Iso)propanol und/oder 2-(Iso)propanol) handeln.

Unter einem Zielgas wird insofern ein Gas oder Gasgemisch verstanden, dessen Konzentration in einer Messumgebung des Gasmessgeräts bestimmt und ausgegeben werden soll. Auch hierbei kann es sich um ein Alkohol (z.B. Ethanol, Methanol, 1-(Iso)propanol und/oder 2-(Iso)propanol) handeln.

Der Fall, dass sowohl das Schadgas als auch das Zielgas ein Alkohol sein können, kann beispielsweise auftreten, wenn das Gasmessgerät als Alkoholmessgerät ausgestaltet ist, welches in einer potenziell alkoholhaltigen Lagerungsumgebung gelagert wird. Alkohole können in der Lagerungsumgebung beispielsweise durch Desinfektionsmittel oder Reinigungsmittel vorhanden sein (z.B. Scheibenwischflüssigkeit oder Cockpit-Reiniger fürs Auto). Ein Alkohol als Schadgas liegt dabei beispielsweise in einer Konzentration im Prozent-Bereich vor, während ein Alkohol als Zielgas dabei beispielsweise in einer Konzentration im Promille-Bereich vorliegen kann.

Bei der Messumgebung kann es sich um eine unmittelbare Umgebung des Gasmessgeräts handeln, beispielsweise um eine das Gasmessgerät umgebende Atmosphäre. Ergänzend oder alternativ kann es sich bei der Messumgebung um eine mittelbare Umgebung des Gasmessgeräts handeln, beispielsweise um ein dem Gasmessgerät durch ein Probenzuführelement zugeführtes Gas oder Gasgemisch, welches von der unmittelbaren Umgebung verschieden sein kann. Im Fall einer Atemgasmessung ist die Messumgebung beispielsweise eine Atemgasprobe, welche dem Gasmessgerät beispielsweise mittels Mundstück oder Trichter zugeführt werden kann.

Unter einer Auswerteeinheit wird eine mittels geeigneter Hardware und/oder Software zur Ausführung von Schritten der Signaleingabe, Datenverarbeitung und Signalausgabe ausgebildete Komponente eines Gasmesssystems verstanden. Die Auswerteeinheit kann dazu eingerichtet sein, einige oder alle Schritte des erfindungsgemäßen Verfahrens auszuführen. Die Auswerteeinheit kann beispielsweise als ein Prozessor oder als ein Mikrokontroller ausgestaltet sein. Die Auswerteeinheit kann als Bauelement des Gasmessgeräts vorliegen oder als eine hiervon getrennte Komponente vorliegen. Wenn das Gasmessgerät und die Auswerteeinheit als physisch getrennte Komponenten vorliegen, ist es bevorzugt, dass diese datentechnisch verbindbar sind, beispielsweise über eine kabellose Schnittstelle.

Das Ausgeben der Warnung kann mittels einer Alarmierungseinheit erfolgen, wobei die Warnung durch optische und/oder akustische Ausgabe erfolgen kann. Das Ausgeben der Warnung im dritten Betriebszustand kann beispielsweise erfolgen, wenn das Gerät in den zweiten Betriebszustand überführt wird oder sich in diesem befindet oder schon während der Lagerung (d.h. im ersten Betriebszustand).

Die Schritte "Lagern des Gasmessgeräts in der Lagerungsumgebung in dem ersten Betriebszustand" und "Bestimmen einer Konzentration eines Schadgases in der Lagerungsumgebung durch eine Auswerteeinheit" erfolgen bevorzugt gleichzeitig, wobei der Schritt "Bestimmen der Konzentration des Zielgases in der Messumgebung durch die Auswerteeinheit in dem zweiten Betriebszustand" unter Berücksichtigung der Konzentration des Schadgases und "Ausgeben der Konzentration des Zielgases", und/oder der Schritt "Vergleichen der mit der Konzentration des Schadgases korrelierenden Information mit dem vorbestimmten Schwellenwert durch die Auswerteeinheit und Ausgeben der Warnung in einem dritten Betriebszustand", wenn die korrelierenden Information den vorbestimmten Schwellenwert überschreitet vorzugsweise zeitlich versetzt dazu erfolgen.

Vorzugsweise wird der Schritt "Bestimmen der Konzentration des Schadgases in der Lagerungsumgebung" wiederholt, insbesondere periodisch wiederholend in dem ersten Betriebszustand durchgeführt.

Auf diese Weise kann in dem ersten Betriebszustand eine Mehrzahl zeitlich aufgelöster Messsignale, welche mit der Konzentration des Schadgases zu den jeweiligen Messzeitpunkten korrelieren, gewonnen und zur (gleichzeitigen oder nachgelagerten) Verarbeitung durch die Auswerteeinheit bereitgestellt werden.

Beispielsweise ist es möglich, in dem ersten Betriebszustand in einem Intervall von beispielsweise 1 Minute regelmäßig, d.h. periodisch wiederholend, Messsignale, welche mit der Konzentration des Schadgases in der Lagerungsumgebung korrelieren, und somit Messwerte der Konzentration des Schadgases bereitzustellen und mittels der Auswerteeinheit auszuwerten.

Vorzugsweise weist das Verfahren ferner den Schritt auf: Bestimmen, ob die Konzentration des Schadgases und/oder eine Menge des Schadgases den vorbestimmten Schwellenwert überschreitet, wobei dieser Verfahrensschritt durch die Auswerteeinheit durchgeführt wird. Ferner vorzugsweise weist das Verfahren den Schritt auf: Ausgeben der Warnung, wenn die Konzentration und/oder Menge des Schadgases den vorbestimmten Schwellenwert überschreitet oder überschritten hat.

Die Konzentration und/oder die Menge des Schadgases sind somit Beispiele für eine mit der Konzentration des Schadgases korrelierenden Information.

Auf diese Weise kann das Ausgeben der Warnung dahingehend angepasst werden, dass überprüft wird, ob eine Konzentration des Schadgases einen vorbestimmten Schwellenwert und/oder ob eine Menge des Schadgases einen vorbestimmten Schwellenwert überschritten hat. Je nach Eigenschaft des zu überwachenden Gasmesssystems kann die Ausgabe einer Warnung an das Messverhaltens das Gasmessgeräts angepasst werden.

Das Ausgeben der Warnung kann unmittelbar dann erfolgen, sobald die Eintrittsbedingung vorliegt oder in einem zeitlich versetzen Schritt, beispielsweise sobald ein Benutzer das Gasmessgeräts eingeschaltet (Übergang von dem ersten Betriebszustand in den zweiten Betriebszustand).

Vorzugsweise ist zur Bestimmung der Konzentration des Schadgases in der Lagerungsumgebung ein erster Gassensor bereitgestellt, wobei zur Bestimmung der Konzentration des Zielgases in der Messumgebung ein zweiter (d.h. separater) Gassensor bereitgestellt ist.

Auf diese Weise können die Messung des Schadgases und die Messung des Zielgases durch separate Gassensoren vorgenommen werden. Das jeweilige Messverhalten der Gassensoren kann insofern jeweils auf die Messung des Schadgases und die Messung des Zielgases, die jeweiligen Umgebungsbedingungen, wie beispielsweise hohe Konzentrationen von Schadgas und niedrige Konzentrationen von Zielgas und ein jeweiliges Probennahmeverfahren optimiert werden. Ferner ist es unter Umständen möglich den Energieverbrauch des Gasmesssystems im zeitlichen Mittel zu senken, da die Überwachung der Lagerungsumgebung lediglich mittels des ersten Gassensors erfolgen kann, während der zweite Gassensor im ersten Betriebszustand nicht betrieben wird.

Es ist bevorzugt, dass der erste Gassensor und/oder der zweite Gassensor jeweils als elektrochemischer Gassensor ausgestaltet ist.

Unter einem elektrochemischen Gassensor wird eine elektrochemische Zelle verstanden, die eingerichtet ist, wenigstens eine gasförmige Substanz, insbesondere das Schadgas und/oder das Zielgas, in einem Gas oder Gasgemisch, insbesondere in der Lagerungsumgebung bzw. Messumgebung, nachzuweisen und eine Anzahl mit der Konzentration der Substanz korrespondierender Messsignale bereitzustellen.

Vorzugsweise umfasst der elektrochemische Gassensor zumindest eine Messelektrode und einen Gegenelektrode, vorzugsweise ferner zumindest eine Referenzelektrode. Das Vorliegen weiterer Elektroden ist möglich. Vorzugsweise umfasst der elektrochemische Gassensor einen sauren, flüssigen Elektrolyten, welcher wenigstens mit einem Teil der Elektroden in Kontakt steht. Der elektrochemische Gassensor kann als Brennstoffzelle ausgestaltet sein.

Besonders bevorzugt ist der erste Gassensor als 3-Elektroden-Sensor ausgestaltet. Ergänzend oder alternativ ist bevorzugt, dass der zweite Gassensor als 2-Elektroden-Sensor ausgestaltet ist. Es ist bevorzugt, dass der 3-Elektroden-Sensor nach einem amperometrischen Messprinzip arbeitet und dass der 2-Elektroden-Sensor nach einem coulometrischem Messprinzip arbeitet.

Es ist bevorzugt, dass der erste Gassensor gegenüber dem Schadgas langzeitbeständig ist und dass der zweite Gassensor gegenüber dem Zielgas besonders sensitiv ist.

Es ist bevorzugt, dass der erste Gassensor zur passiven Probennahme eingerichtet ist und/oder dass der zweite Gassensor zur aktiven Probennahme, beispielsweise mittels Pumpenvorrichtung, eingerichtet ist.

Wenn einer der beiden Gassensoren als 3-Elektroden-Sensor ausgestaltet ist, ist bevorzugt, dass das Gasmessgerät ferner eine Potentiostatenschaltung aufweist.

Das Gasmessgerät kann weitere Komponenten aufweisen, beispielsweise ein Probennahmesystem umfassend einen Hubmagneten und einen Faltenbalg.

Vorzugsweise umfasst der Schritt Bestimmen der Konzentration des Zielgases in der Messumgebung in dem zweiten Betriebszustand unter Berücksichtigung der Konzentration des Schadgases den Schritt Korrektur einer Empfindlichkeit des zweiten Gassensors durch die Auswerteeinheit.

Auf diese Weise kann besonders einfach eine korrigierte Konzentration des Zielgases ausgegeben werden.

Vorzugsweise umfasst der Schritt Korrektur der Empfindlichkeit des zweiten Gassensors den zusätzlichen Schritt Bestimmung eines aktuellen Vergiftungszustands und/oder eines aktuellen Erholungszustands des zweiten Gassensors durch die Auswerteeinheit.

Auf diese Weise kann der dynamische Einfluss des Schadgases auf das Messverhalten des zweiten Gassensors berücksichtigt werden.

Der Vergiftungszustand bezeichnet dabei einen sich negativ auf die Empfindlichkeit des zweiten Gassensors auswirkenden Einfluss des Schadgases. Der Erholungszustand bezeichnet dabei den Rückgang des Einflusses des Schadgases auf die Empfindlichkeit des zweiten Gassensors.

Es ist besonders bevorzugt, sowohl den Vergiftungszustand als auch den Erholungszustand zu berücksichtigen, da es möglich ist, dass das dynamische Verhalten des Vergiftungszustands und das dynamische Verhalten des Erholungszustands unterschiedliche Zeitkonstanten aufweisen.

Erfindungsgemäß wird ferner ein Gasmesssystem bereitgestellt.

Das Gasmesssystem weist einen ersten Gassensor auf, welcher eingerichtet ist, ein mit einer Konzentration eines Schadgases in einer Lagerungsumgebung des ersten Gassensors vorhandenen Gases korrelierendes erstes Messsignal bereitzustellen. Das Gasmesssystem weist ferner einen zweiten Gassensor auf, welcher eingerichtet ist, ein mit einer Konzentration eines Zielgases in einer Messumgebung des zweiten Gassensors vorhandenen Gases korrelierendes zweites Messsignal bereitzustellen. Das Gasmesssystem weist ferner eine Auswerteeinheit auf, welche eingerichtet ist, das erste Messsignal und das zweite Messsignal zu empfangen und einige oder alle Schritte des vorbeschriebenen erfindungsgemäßen Verfahrens auszuführen.

Das Gasmesssystem weist zu dem beschriebenen erfindungsgemäßen Verfahren vergleichbare Vorteile und Wirkungen auf. Alle im Zusammenhang mit dem Verfahren offenbarten Merkmale und vorzugsweisen Ausgestaltungen gelten ebenso als im Zusammenhang mit dem Gasmesssystem offenbart und umgekehrt.

Der erste Gassensor, der zweite Gassensor und die Auswerteeinheit können in einem gemeinsamen Gerät bereitgestellt sein oder in verschiedenen Geräten. Beispielsweise können der erste Gassensor und die Auswerteeinheit in einem ersten Gerät und der zweite Gassensor in einem zweiten Gerät bereitgestellt sein. In einem anderen Beispiel können der zweite Gassensor und die Auswerteeinheit in einem ersten Gerät und der erste Gassensor in einem zweiten Gerät bereitgestellt sein. In einem weiteren Beispiel können der zweite Gassensor und die Auswerteeinheit in einem ersten Gerät und der erste Gassensor und eine weitere oder ergänzende Auswerteeinheit in einem zweiten Gerät bereitgestellt sein.

Vorzugsweise weist das Gasmesssystem ein Alkoholmessgerät auf, welches eingerichtet ist, eine Konzentration von Alkohol als Zielgas in einer Messumgebung des Alkoholmessgeräts, insbesondere in einer Atemgasprobe zu bestimmen, wobei das Alkoholmessgerät den zweiten Gassensor aufweist. Das Alkoholmessgerät kann darüber hinaus den ersten Gassensor aufweisen, wobei dies nicht erforderlich ist. Ebenso kann das Alkoholmessgerät die Auswerteeinheit aufweisen, wobei auch dies nicht erforderlich ist.

Beispielsweise kann es sich bei dem Alkoholmessgerät um ein Atemalkoholmessgerät handeln.

Das Alkoholmessgerät, insbesondere das Atemalkoholmessgerät, kann ein aktives Probennahmesystem wie eine Pumpenvorrichtung oder Pumpeneinheit aufweisen, um dem zweiten Gassensor ein vorzugsweise definiertes Volumen einer Atemgasprobe zuzuführen und die Konzentration von Alkohol als Zielgas in der Messumgebung, insbesondere in der Atemgasprobe bestimmen.

Vorzugsweise weist das Gasmesssystem ein Überwachungsgerät auf, welches eingerichtet ist, die Konzentration des Schadgases in der Lagerungsumgebung zu bestimmen. Dabei weist das Überwachungsgerät den ersten Gassensor auf. Ein Überwachungsgerät ist also ein Gerät, welches den ersten Gassensor zur Überwachung der Konzentration des Schadgases in der Lagerungsumgebung aufweist.

In dieser bevorzugten Ausgestaltung umfasst das Gasmesssystem also mindestens zwei getrennte Geräte, nämlich das Alkoholmessgerät und das Überwachungsgerät. Es ist bevorzugt, dass das Überwachungsgerät ferner die Auswerteeinheit aufweist, wobei dies nicht erforderlich ist. Das Alkoholmessgerät und das Überwachungsgerät können über eine datentechnische Schnittstelle, beispielsweise über eine kabellose Verbindung, datentechnisch verbindbar sein.

Auf diese Weise kann ein erfindungsgemäßes Gasmesssystem durch Nachrüsten eines bekannten Alkoholmessgeräts bereitgestellt werden, indem das Überwachungsgerät als zusätzliche Komponente des Gasmesssystems bereitgestellt wird. Die Funktionalität bestehender Alkoholmessgerät kann somit verbessert werden.

Vorzugsweise weist das Alkoholmessgerät und/oder das Überwachungsgerät eine Alarmierungseinheit zur optischen und/oder akustischen Ausgabe der Warnung auf. Die Alarmierungseinheit kann beispielsweise als Display und/oder als Lautsprecher ausgestaltet sein. Es ist bevorzugt, dass die Alarmierungseinheit als Bauelement des Überwachungsgerät ausgestaltet ist, dies ist jedoch nicht erforderlich.

Alle hierin offenbarten Merkmale sind beliebig miteinander kombinierbar, soweit dies nicht Alternativen betrifft oder widersprüchlich ist.

Diese und weitere Merkmale und vorteilhafte Ausgestaltungen der Erfindung ergeben sich auch aus der nachfolgenden Figurenbeschreibung. Dabei zeigt:

Fig. 1     ein Ausführungsbeispiel eines schematisch dargestellten erfindungsgemäßen Gasmesssystems,

Fig. 2     ein weiteres Ausführungsbeispiel eines schematisch dargestellten erfindungsgemäßen Gasmesssystems,

Fig. 3     ein schematisches Ablaufdiagramm nach einem Ausführungsbeispiel eines erfindungsgemäßen Verfahrens,

Fig. 4     ein schematisches Ablaufdiagramm nach einem weiteren Ausführungsbeispiel eines erfindungsgemäßen Verfahrens,

Fig. 5     ein schematisches Ablaufdiagramm nach noch einem weiteren Ausführungsbeispiel eines erfindungsgemäßen Verfahrens,

Fig. 6     zeitliche Verläufe einer Konzentration von Schadgas in einer Lagerungsumgebung und eines Vergiftungszustands.

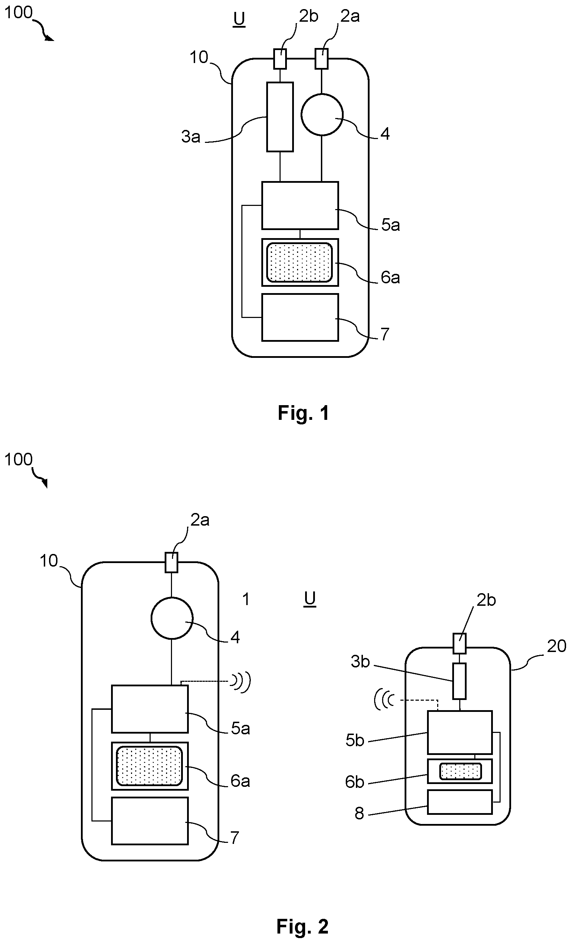

Erfindungsgemäß wird zunächst ein Gasmesssystem 100 bereitgestellt. Zwei Ausführungsbeispiele erfindungsgemäßer Gasmesssysteme 100 sind in Fig. 1 und 2 dargestellt.

Jedes erfindungsgemäße Gasmesssystem 100 weist einen ersten Gassensor 3a, 3b auf, welcher eingerichtet ist, ein mit einer Konzentration eines Schadgases cS in einer Lagerungsumgebung U des ersten Gassensors 3a, 3b vorhandenen Gases korrelierendes erstes Messignal bereitzustellen. Jedes erfindungsgemäße Gassystem 100 weist ferner einen zweiten Gassensor 4 auf, welcher eingerichtet ist, ein mit einer Konzentration eines Zielgases cZ in einer Messumgebung U des zweiten Gassensors 4 vorhandenen Gases korrelierendes zweites Messignal bereitzustellen. Ferner weist jedes erfindungsgemäße Gasmesssystem 100 eine Auswerteeinheit 5a, 5b auf, welche eingerichtet ist, das erste Messignal und das zweite Messignal zu empfangen und einige oder alle Schritte S1, S2, ... des noch zu beschreibenden erfindungsgemäßen Verfahrens 200 auszuführen.

Insofern im Folgenden lediglich das Gasmesssystem 100 referenziert wird, gelten in diesem Zusammenhang offenbarte Merkmale und Wirkungen für jede mögliche Ausführungsform eines erfindungsgemäßen Gasmesssystems 100.

Der erste Gassensor 3a, 3b, der zweite Gassensor 4 und die Auswerteeinheit 5a, 5b können als Elemente eines gemeinsamen Gerätes ausgestaltet sein. Dies ist dem Ausführungsbeispiel nach Fig. 1 der Fall, in welchem ein Gasmessgerät 10 bereitgestellt wird, welches die genannten Komponenten aufweist. Die Gassensoren 3a, 4 stehen über entsprechende Öffnungen 2a, 2b in strömungstechnischer Verbindung mit der Umgebung U, welche je nach Betriebszustand die Lagerungsumgebung U oder die Messumgebung U sein kann. Über die Öffnungen 2a, 2b können der erste Gassensor 3b und/oder der zweite Gassensor 4 im Diffusionsbetrieb arbeiten. Ergänzend oder alternativ können der erste Gassensor 3b und/oder der zweite Gassensor 4 zur aktiven Probennahme über die Öffnungen 2a, 2b eingerichtet sein.

Die Messumgebung U kann dabei auch mittelbar durch ein Probenzuführelement wie ein Mundstück oder einen Trichter zum zweiten Gassensor 4 geführt werden, so dass die Messumgebung U auch durch eine Atemgasprobe eines Probanden bereitgestellt sein kann. Hierzu kann das Gasmessgerät 10 eine Schnittstelle zur Aufnahme eines Probenzuführelements aufweisen, welches eine strömungstechnische Verbindung zwischen Öffnung 2a und einem Mund eines Probanden bereitstellen kann.

Es ist auch möglich, dass der erste Gassensor 3b, der zweite Gassensor 4 und die Auswerteeinheit 5a, 5b Elemente unterschiedlicher Geräte des Gasmesssystems 100 sein können. Ein Beispiel eines solchen Falls ist in Fig. 2 dargestellt. In diesem Ausführungsbeispiel ist der zweite Gassensor 4 in einem Gasmessgerät 10 bereitgestellt, während der erste Gassensor 3b und die Auswerteeinheit 5b Elemente eines Überwachungsgeräts 20 sind. Das Gasmessgerät 10 dieses Ausführungsbeispiels weist ebenfalls eine Auswerteeinheit 5a auf, so dass die beiden Auswerteeinheiten 5a, 5b unterschiedliche oder gleiche Funktionen ausführen können.

Jedes Gasmessgerät 10 kann als Alkoholmessgerät 10 ausgestaltet sein, welches eingerichtet ist, eine Konzentration von Alkohol als Zielgas in einer Messumgebung U des Alkoholmessgeräts 10, insbesondere in einer Atemgasprobe zu bestimmen, wobei das Alkoholmessgerät 10 wie beschrieben den zweiten Gassensor 4 aufweist.

Das Gasmesssystem 100, insbesondere jede Komponente des Gasmesssystems 100, kann weitere Elemente aufweisen. Im Ausführungsbeispiel nach Fig. 1 weist beispielsweise das Gasmessgerät 10 ferner einen Energiespeicher 7 zur Versorgung der elektrischen und/oder elektronischen Komponenten des Gasmessgeräts 10 mit Energie auf. Ebenso weisen das Gasmessgerät 10 und das Überwachungsgerät 20 im Ausführungsbeispiel nach Fig. 2 einen entsprechenden Energiespeicher 7 auf.

Es ist bevorzugt und in den Ausführungsbeispielen nach Fig. 1 und Fig. 2 dargestellt, dass das Gasmesssystem 100, insbesondere das Gasmessgerät 10, beispielsweise das Alkoholmessgerät 10, und/oder das Überwachungsgerät 20 eine Alarmierungseinheit 6a, 6b zu optischen und/oder akustischen Ausgabe der Warnung aufweisen kann. Die Alarmierungseinheit 6a, 6b kann beispielsweise als Bildschirm ausgestaltet sein.

Wenn die Komponenten des Gasmesssystems 100 nicht in einem gemeinsamen Gasmessgerät 10 aufgenommen sind, ist es bevorzugt, dass eine datentechnische Verbindung zwischen den Geräten 10, 20 des Gasmesssystems 100 bereitgestellt werden kann. Dies ist in Fig. 2 als Funkverbindung schematisch angedeutet. Entsprechendes gilt, wenn die Auswerteeinheit 5a, 5b - was nicht dargestellt, aber möglich ist - als weitere von den Geräten 10, 20 physisch getrennte Komponente des Gasmesssystems 100 ausgestaltet ist.

Bei dem ersten Gassensor 3a, 3b und dem zweiten Gassensor 4 kann es sich jeweils um einen elektrochemischen Gassensor handeln.

Wenn das Gasmesssystem 100 durch mehrere Geräte 10, 20 realisiert ist, ist es bevorzugt, dass lediglich eines der Geräte 20 die Auswerteeinheit 5a, 5b aufweist. Es ist jedoch möglich, dass jedes der Geräte 10, 20 eine (redundant oder sich ergänzend ausgestaltete) erfindungsgemäße Auswerteeinheit 5a, 5b aufweist.

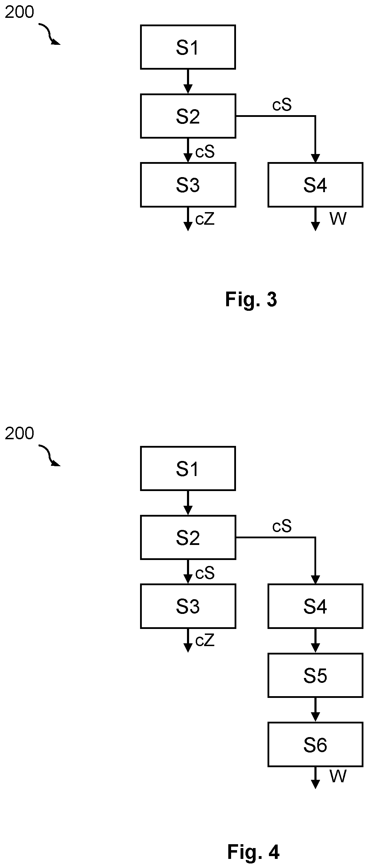

Schematische Ablaufdiagramme erfindungsgemäßer Verfahren 200 sind in Fig. 3, 4 und 5 dargestellt. Jedes erfindungsgemäße Verfahren 200 kann zum Betreiben eines vorbeschriebenen Gasmesssystems 100 dienen.

Insofern im Folgenden lediglich das Verfahren 200 referenziert wird, gilt dies für jede mögliche Ausführungsform eines erfindungsgemäßen Verfahren 200 gleichermaßen.

Das Verfahren 200 weist den Schritt S1: Lagern eines Gasmessgeräts 10 in einer Lagerungsumgebung U in einem ersten Betriebszustand auf. Der erste Betriebszustand kann beispielsweise ein Zustand sein, in welchem eine Gasmessung durch den zweiten Gassensor 4 nicht stattfindet, jedoch eine Gasmessung durch den ersten Gassensor 3a, 3b stattfindet.

Das Verfahren 200 weist den Schritt S2: Bestimmen einer Konzentration eines Schadgases in der Lagerungsumgebung U durch eine Auswerteeinheit 5a, 5b auf. Hierzu kann die Auswerteeinheit 5a, 5b in (quasi-)Echtzeit oder zeitlich versetzt, beispielsweise durch Abrufen von zwischengespeicherten Daten, das oder die Messsignale des ersten Gassensors 3a, 3b verwenden. Schritt S2 kann wiederholt, insbesondere im ersten Betriebszustand periodisch wiederholend durchgeführt werden.

Das Verfahren 200 weist den Schritt S3: Bestimmen einer Konzentration des Zielgases cZ in einer Messumgebung U in einem zweiten Betriebszustand durch die Auswerteeinheit 5a, 5b unter Berücksichtigung der Konzentration des Schadgases cS und Ausgeben der Konzentration des Zielgases cZ auf. Schritt S3 erfolgt vorzugsweise zeitlich versetzt zu Schritt S2, d. h. zeitlich nach Schritt S2. Der zweite Betriebszustand kann beispielsweise ein Zustand sein, in welchem die Gasmessung durch den zweiten Gassensor 4 stattfindet.

Das Verfahren 200 weist ergänzend oder alternativ zu Schritt S3 den Schritt S4: Vergleichen einer mit der Konzentration des Schadgases cS korrelierenden Information mit einem vorbestimmten Schwellenwert durch die Auswerteeinheit 5a, 5b und Ausgeben einer Warnung in einem dritten Betriebszustand, wenn die korrelierenden Informationen den vorbestimmten Schwellenwert überschreitet auf. Schritt S4 kann dabei im Wesentlichen sofort ausgeführt werden, wenn das Überschreiten des vorbestimmten Schwellenwerts eintritt oder dazu zeitlich nachgelagert, beispielsweise abhängig von weiteren Bedingungen erfolgen. Beispielsweise kann Schritt S4 ausgeführt werden, wenn das Gasmessgerät 10 eingeschaltet, d.h. von dem ersten Betriebszustand in den zweiten Betriebszustand überführt wird, um einen dann sicher anwesenden Benutzer entsprechend zu warnen.

Das erfindungsgemäße Verfahren 200 kann wie in Fig. 4 dargestellt ferner optional Schritte S5 und S6 aufweisen.

Schritt S5 ist das Bestimmen, durch die Auswerteeinheit 5a, 5b, ob die Konzentration und/oder eine Menge des Schadgases den vorbestimmten Schwellenwert überschritten hat.

Schritt S6 ist das Ausgeben der Warnung, wenn die Konzentration und/oder die Menge des Schadgases den vorbestimmten Schwellenwert überschritten hat.

Schritt S6 kann im Wesentlichen zeitgleich mit Schritt S5 ausgeführt werden oder dazu zeitlich nachgelagert, beispielsweise wenn das Gasmessgerät 10 eingeschaltet, d.h. von dem ersten Betriebszustand in den zweiten Betriebszustand überführt wird, um einen dann sicher anwesenden Benutzer entsprechend zu warnen.

Beispielsweise kann die Bestimmung, ob die Menge des Schadgases den vorbestimmten Schwellenwert überschritten hat entsprechend nachstehender Formel erfolgen:

$$\sum_{i=-N}^{0} a(T) \cdot cS(t - i \cdot \Delta t) = \begin{cases} < g \rightarrow nicht\ \ddot{u}berschritten \\ \geq g \rightarrow \ddot{U}berschritten \end{cases}$$

wobei g der vorbestimmte Schwellenwert ist, cS die Konzentration des Schadgases, $\Delta t$ eine Abtastzeit der Messwerte, N eine Anzahl der zu berücksichtigenden Messwerte und a(T) eine optionale von der Temperatur T abhängige Gewichtsfunktion ist.

In einem weiteren Beispiel kann die Bestimmung durch die Auswerteeinheit 5a, 5b auch anhand eines rekursiven Tiefpassfilters erfolgen. In diesem Beispiel dienen die Messwerte für die Konzentration des Schadgases cS als Eingang des Tiefpassfilters, wobei das Ausgangssignal des Tiefpassfilters mit dem vorbestimmten Grenzwert verglichen wird.

Es ist bevorzugt, dass bei dem erfindungsgemäßen Verfahren 200 zur Bestimmung der Konzentration des Schadgases cS in der Lagerungsumgebung U ein erster Gassensor 3a, 3b bereitgestellt ist, wobei zur Bestimmung der Konzentration des Zielgases cZ in der Messumgebung U ein zweiter Gassensor 4 bereitgestellt ist, wie dies im Zusammenhang mit dem erfindungsgemäßen Gasmesssystem 100 beschrieben worden ist.

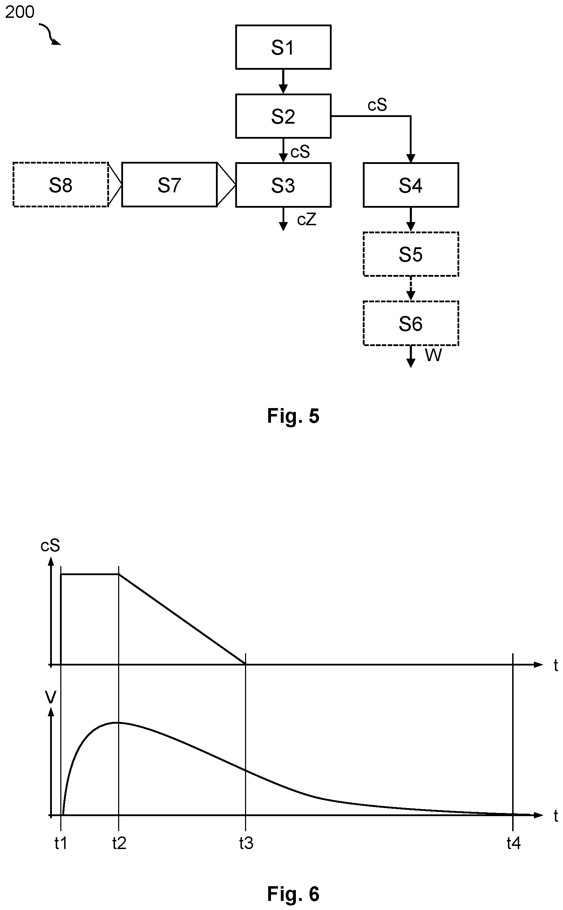

Es ist bevorzugt und in dem Ausführungsbeispiel nach Fig. 5 dargestellt, dass der Schritt S3 den Schritt S7: Korrektur einer Empfindlichkeit des zweiten Gassensors 4 durch die Auswerteeinheit 5a, 5b umfasst. Der Schritt S7 kann insofern als zusätzlicher Schritt in jedem der Ausführungsbeispiele nach Fig. 3 und 4 vorhanden sein.

Insofern ist es bekannt, eine Zuordnung eines Messsignals eines Gassensors zu einem entsprechenden Wert für eine Konzentration des gemessenen Gases (Zielgas und/oder Schadgas) durch Verwendung eines entsprechenden Proportionalfaktors, zum Beispiel eines Justierfaktors, vorzunehmen. Durch Anpassung der Empfindlichkeit wird effektiv eine Veränderung des Proportionalfaktors erreicht, sodass das durch das Schadgas veränderte Messverhalten des zweiten Gassensors 4 entsprechend berücksichtigt werden kann.

Es ist bevorzugt und ebenfalls in dem Ausführungsbeispiel nach Fig. 5 dargestellt, dass der Schritt S7 optional den weiteren Schritt S8: Bestimmung eines aktuellen Vergiftungszustands V und/oder eines aktuellen Erholungszustands des zweiten Gassensors 4 durch die Auswerteeinheit 5a, 5b umfassen kann.

Beispielsweise können für die Korrektur der Empfindlichkeit sowohl der Vergiftungszustand V als auch der Erholungszustand modelliert werden, d. h. das dynamische Verhalten von Vergiftung und Erholung. Die Bestimmung des Vergiftungszustands V kann anhand der Konzentration cS und/oder Menge des Schadgases erfolgen.

Der Vergiftungszustand V kann zum Beispiel mit einem modifizierten Modell erster Ordnung modelliert werden. Durch die Modifizierung ist es möglich, ein Fortschreiten der Vergiftung nur dann zu berücksichtigen, wenn die aktuelle Konzentration und/oder Menge des Schadgases cS den aktuellen Vergiftungswert V überschreitet. Solange diese Bedingung nicht erfüllt ist, findet eine Erholung statt, wobei davon ausgegangen wird, dass eine Zeitkonstante der Erholung $\tau rec$ kleiner ist als eine Zeitkonstante der Vergiftung $\tau cont$.

Es ist beispielsweise mit einem linearen Differenzialgleichungs-System erster Ordnung möglich, einen gekoppelten Vergiftung-Erholung-Prozess zu modellieren, beispielsweise gemäß nachstehendem System:

$$\tau cont \cdot \frac{\partial V(t)}{\partial t} + V(t) = K \cdot c(t) \qquad \text{für } K \cdot c(t) \geq V(t)$$

$$\tau rec \cdot \frac{\partial V(t)}{\partial t} + V(t) = K \cdot c(t) \qquad \text{für } K \cdot c(t) < V(t)$$

Wobei $\tau cont$ die Zeitkonstante für den Vergiftungsprozess dargestellt, $\tau rec$ die Zeitkonstante für den Erholungsprozess, V(t) den Vergiftungszustand zum Zeitpunkt t, c(t) die Konzentration des Schadgases cS zum Zeitpunkt t und K eine Konstante, welche das Verhältnis von Vergiftungsgrad V(t) zu der Konzentration des Schadgases cS(t) im eingeschwungenen Zustand definiert.

Für den Fall, dass die Konzentration des Schadgases cS nicht kontinuierlich bestimmt wird, sondern zu diskreten Abtastzeitpunkten mit einem zeitlichen Abstand (Abtastintervall) $\Delta t$, ist eine Diskretisierung des Differenzialgleichungs-Systems und dessen numerische Lösung möglich. Eine Diskretisierung ist beispielsweise mittels des Vorwärts-Euler-Verfahrens oder mittels des Runge-Kutta-Verfahrens möglich.

Das Modell kann dahingehend modifiziert werden, dass die Zeitkonstante für den Vergiftungsprozess $\tau cont$ und die Zeitkonstante für den Erholungsprozess $\tau rec$ zusätzlich in Abhängigkeit von der aktuellen Temperatur modelliert werden.

Durch Berücksichtigung des aktuellen Vergiftungszustands V und/oder des aktuellen Erholungszustands ist es besonders vorteilhaft möglich, die Empfindlichkeit des zweiten Gassensors 4 numerisch zu korrigieren. Es lässt sich zum Beispiel ein Korrekturfaktor als Funktion des aktuellen Vergiftungszustands V und/oder als Funktion des aktuellen Erholungszustands bestimmen, welcher mit dem vorbeschriebenen Proportionalfaktor multipliziert werden kann.

Die Bestimmung des Korrekturfaktors als Funktion des aktuellen Vergiftungszustands V und/oder als Funktion des aktuellen Erholungszustands kann beispielsweise empirisch erfolgen, indem in einem Versuch bestimmte stationäre Vergiftungszustände V eingestellt werden und der daraus resultierende Einfluss auf die Empfindlichkeit des zweiten Gassensors 4 bestimmt wird. Die so erhaltene Rechenvorschrift kann dann entweder als analytischer Ausdruck angegeben oder beispielsweise in einer Look-up-Tabelle zum Abrufen durch die Auswerteeinheit 5a, 5b hinterlegt werden.

In Fig. 6 ist insofern ein Beispiel eines Verlaufs einer Konzentration von Schadgas cS in einer Lagerungsumgebung U über die Zeit t in einem erfindungsgemäßen Gasmesssystem 100 sowie ein entsprechender mit dem erfindungsgemäßen Verfahren 200 bestimmter zeitlicher Verlauf eines Vergiftungszustands V dargestellt.

Im oberen Diagramm nach Fig. 6 ist ersichtlich, dass in dem Beispiel in einer Lagerungsumgebung U die Konzentration des Schadgases cS zu einem Zeitpunkt t1 sprungartig auf ein Maximum ansteigt, welches bis zum Zeitpunkt t2 erhalten bleibt, bevor es bis zu einem Zeitpunkt t3 rampenartig wieder auf Null absinkt. Die nach dem erfindungsgemäßen Verfahren 200 bestimmte aktuelle Vergiftung V über die Zeit t ist im unteren Diagramm nach Fig. 6 aufgetragen. Es ist ersichtlich, dass beginnend mit dem Zeitpunkt t1 bis zum Zeitpunkt t2 die berechnete Vergiftung V(t) kontinuierlich ansteigt und dann über den Zeitpunkt t3 hinaus bis etwa zum Zeitpunkt t4 erst langsam wieder auf Null absinkt.

Bezugszeichenliste

| 2a, 2b | Öffnungen |
|---|---|
| 3a, 3b | erster Gassensor |
| 4 | zweiter Gassensor |
| 5a, 5b | Auswerteeinheit |
| 6a, 6b | Alarmierungseinheit |
| 7 | Energiespeicher |
| 10 | Gasmessgerät, Alkoholmessgerät |
| 20 | Überwachungsgerät |
| 100 | Gasmesssystem |
| 200 | Verfahren |

| cS | Konzentration des Schadgases |
|---|---|
| cZ | Konzentration des Zielgases |
| g | vorbestimmter Schwellenwert |
| S1, S2, ... | Verfahrensschritte |
| t | Zeit |
| t1, t2 | Zeitpunkte |
| U | Umgebung, Lagerungsumgebung, Messumgebung |
| V | Vergiftungszustand, Vergiftungswert |
| W | Warnung |

**Patentansprüche**

1. Verfahren (200) zum Betreiben eines Gasmesssystems (100), aufweisend die Schritte:

- (S1) Lagern eines Gasmessgeräts (10) in einer Lagerungsumgebung (U) in einem ersten Betriebszustand, und
- (S2) Bestimmen einer Konzentration eines Schadgases (cS) in der Lagerungsumgebung (U) durch eine Auswerteeinheit (5a, 5b); und
- (S3) Bestimmen einer Konzentration eines Zielgases (cZ) in einer Messumgebung (U) durch die Auswerteeinheit (5a, 5b) in einem zweiten Betriebszustand unter Berücksichtigung der Konzentration des Schadgases (cS) und Ausgeben der Konzentration des Zielgases (cZ), und/oder
- (S4) Vergleichen einer mit der Konzentration des Schadgases (cS) korrelierenden Information mit einem vorbestimmten Schwellenwert (g) durch die Auswerteeinheit (5a, 5b) und Ausgeben einer Warnung (W) in einem dritten Betriebszustand, wenn die korrelierende Information den vorbestimmten Schwellenwert (g) überschreitet,

wobei der erste Betriebszustand und der zweite Betriebszustand zeitlich versetzt vorliegen.

2. Verfahren (200) nach Anspruch 1,
wobei der Schritt (S2) Bestimmen der Konzentration des Schadgases (cS) in der Lagerungsumgebung (U) wiederholt durchgeführt wird.

3. Verfahren (200) nach Anspruch 2,
ferner aufweisend den Schritt:

- (S5) Bestimmen, durch die Auswerteeinheit (5a, 5b), ob die Konzentration (cS) und/oder eine Menge des Schadgases den vorbestimmten Schwellenwert (g) überschritten hat, und
- (S6) Ausgeben der Warnung (W), wenn die Konzentration (cS) und/oder Menge des Schadgases den vorbestimmten Schwellenwert (g) überschritten hat.

4. Verfahren (200) nach einem der vorherigen Ansprüche,

wobei zur Bestimmung der Konzentration des Schadgases (cS) in der Lagerungsumgebung (U) ein erster Gassensor (3a, 3b) bereitgestellt ist, und
wobei zur Bestimmung der Konzentration des Zielgases (cZ) in der Messumgebung (U) ein zweiter Gassensor (4) bereitgestellt ist.

5. Verfahren (200) nach Anspruch 4,
wobei der Schritt (S3) Bestimmen der Konzentration des Zielgases (cZ) in der Messumgebung (U) in dem zweiten Betriebszustand unter Berücksichtigung der Konzentration des Schadgases (cS) den Schritt (S7) Korrektur einer Empfindlichkeit des zweiten Gassensors (4) durch die Auswerteeinheit (5a, 5b) umfasst.

6. Verfahren (200) nach Anspruch 5,
   wobei der Schritt (S7) Korrektur der Empfindlichkeit des zweiten Gassensors (4) den zusätzlichen Schritt (S8) Bestimmung eines aktuellen Vergiftungszustands (V) und/oder eines aktuellen Erholungszustands des zweiten Gassensors (4) durch die Auswerteeinheit (5a, 5b) umfasst.

7. Gasmesssystem (100), aufweisend:

   - einen ersten Gassensor (3a, 3b), welcher eingerichtet ist, ein mit einer Konzentration eines Schadgases (cS) in einer Lagerungsumgebung (U) des ersten Gassensors (3a, 3b) vorhandenen Gases korrelierendes erstes Messignal bereitzustellen,
   - einen zweiten Gassensor (4), welcher eingerichtet ist, ein mit einer Konzentration eines Zielgases (cZ) in einer Messumgebung (U) des zweiten Gassensors (4) vorhandenen Gases korrelierendes zweites Messignal bereitzustellen, und
   - eine Auswerteeinheit (5a, 5b), welche eingerichtet ist, das erste Messignal und das zweite Messignal zu empfangen und einige oder alle Schritte des Verfahrens (200) nach einem der Ansprüche 1 bis 6 auszuführen.

8. Gasmesssystem (100) nach Anspruch 7, aufweisend:

   - ein Alkoholmessgerät (10), welches eingerichtet ist, eine Konzentration von Alkohol als Zielgas in der Messumgebung (U) des Alkoholmessgeräts (10), insbesondere in einer Atemgasprobe, zu bestimmen,

   wobei das Alkoholmessgerät (10) den zweiten Gassensor (4) aufweist.

9. Gasmesssystem (100) nach Anspruch 8, aufweisend:

   - ein Überwachungsgerät (20), welches eingerichtet ist, die Konzentration des Schadgases (cS) in der Lagerungsumgebung (U) zu bestimmen, wobei das Überwachungsgerät (20) den ersten Gassensor (3) aufweist.

10. Gasmesssystem (100) nach Anspruch 8 oder 9,
    wobei das Alkoholmessgerät (10) und/oder das Überwachungsgerät (20) eine Alarmierungseinheit (6a, 6b) zur optischen und/oder akustischen Ausgabe der Warnung (W) aufweist.

**Fig. 1**

**Fig. 2**

200

```
      ┌──────────┐
      │    S1    │
      └────┬─────┘
           │
           ▼
      ┌──────────┐      cS
      │    S2    ├───────────┐
      └────┬─────┘           │
           │ cS              ▼
      ┌────▼─────┐      ┌──────────┐
      │    S3    │      │    S4    │
      └────┬─────┘      └────┬─────┘
           │ cZ              │ W
           ▼                 ▼
```

**Fig. 3**

200

```
      ┌──────────┐
      │    S1    │
      └────┬─────┘
           │
           ▼
      ┌──────────┐      cS
      │    S2    ├───────────┐
      └────┬─────┘           │
           │ cS              ▼
      ┌────▼─────┐      ┌──────────┐
      │    S3    │      │    S4    │
      └────┬─────┘      └────┬─────┘
           │ cZ              │
           ▼                 ▼
                        ┌──────────┐
                        │    S5    │
                        └────┬─────┘
                             │
                             ▼
                        ┌──────────┐
                        │    S6    │
                        └────┬─────┘
                             │ W
                             ▼
```

**Fig. 4**

**Fig. 5**

**Fig. 6**

Europäisches
Patentamt

European
Patent Office

Office européen
des brevets

**EUROPÄISCHER RECHERCHENBERICHT**

Nummer der Anmeldung

EP 25 16 1205

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2021/148892 A1 (RULAND SCOTT [US] ET AL) 20. Mai 2021 (2021-05-20)<br>* Absatz [0046]; Abbildung 5 *<br>* Absatz [0073] *<br>* Absatz [0058] *<br>* Absatz [0076] *<br>- - - - - | 1-10 | INV.<br>G01N33/00<br>G01N33/497 |

RECHERCHIERTE
SACHGEBIETE (IPC)

G01N
A61B
B60K

Der vorliegende Recherchenbericht wurde für alle Patentansprüche erstellt

| Recherchenort | Abschlußdatum der Recherche | Prüfer |
|---|---|---|
| Den Haag | 9. Juli 2025 | Mauritz, Jakob |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
Dokument

**ANHANG ZUM EUROPÄISCHEN RECHERCHENBERICHT**
**ÜBER DIE EUROPÄISCHE PATENTANMELDUNG NR.**

EP 25 16 1205

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-07-2025

| Im Recherchenbericht angeführtes Patentdokument | Datum der Veröffentlichung | Mitglied(er) der Patentfamilie | Datum der Veröffentlichung |
|---|---|---|---|
| US 2021148892 A1 | 20-05-2021 | CA 3020501 A1 | 12-04-2019 |
| | | US 2019113502 A1 | 18-04-2019 |
| | | US 2021148892 A1 | 20-05-2021 |
| | | US 2024133868 A1 | 25-04-2024 |
| | | US 2025123266 A1 | 17-04-2025 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 102022108432 A1
- US 20240013647 A

- US A1 A